(19) Europäisches Patentamt
European Patent Office
Office européen des brevets

(11) EP 0 439 699 B1

(12) EUROPÄISCHE PATENTSCHRIFT

(45) Veröffentlichungstag und Bekanntmachung des Hinweises auf die Patenterteilung:
**09.10.1996 Patentblatt 1996/41**

(51) Int. Cl.$^6$: **A01N 33/00**, C08G 73/04

(21) Anmeldenummer: **90121255.5**

(22) Anmeldetag: **07.11.1990**

# (54) Lösungen polymerer Guanidinsalze mit erhöhter Biozidwirksamkeit, Verfahren zu ihrer Herstellung und Verwendung

Solutions of polymeric guanidine salts with increased biocidic activity, process for their preparation and use

Solutions de sels de guanidine polymériques ayant une activité biocidique améliorée, leur procédé de préparation et utilisation

(84) Benannte Vertragsstaaten:
**BE DE FR GB LU NL**

(30) Priorität: **27.01.1990 DE 4002404**

(43) Veröffentlichungstag der Anmeldung:
**07.08.1991 Patentblatt 1991/32**

(73) Patentinhaber: **Degussa Aktiengesellschaft**
**60311 Frankfurt (DE)**

(72) Erfinder:
- **Werle, Peter, Dr.**
  **W-6460 Gelnhausen 2 (DE)**
- **Trageser, Martin**
  **W-6460 Gelnhausen (DE)**
- **Stober, Reinhard, Dr.**
  **W-6467 Hasselroth 2 (DE)**

(56) Entgegenhaltungen:
**EP-A- 0 338 430**
**DE-A- 3 237 074**
**DE-A- 3 635 459**
**GB-A- 1 531 717**
**US-A- 2 325 586**
**US-A- 2 336 605**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.13.7/3.4

**Beschreibung**

Die Erfindung betrifft Lösungen polymerer Guanidinsalze mit erhöhter Biozidwirksamkeit, ein Verfahren zu ihrer Herstellung und Verwendung. Das Verfahren beruht auf der Umsetzung eines Di- oder Polyamins mit Chlorcyan mit nachfolgender Polymerisation. Die Lösungen der polymeren Guanidinsalze können zur Hestellung biozider Mittel Verwendung finden.

Aus der US-PS 2,325,586 sind polymere Guanidine und Salze davon sowie Verfahren zur ihrer Herstellung bekannt. Die polymeren Guanidinsalze lassen sich aus Diaminen durch Umsetzung mit einem Halogencyan mit nachfolgender Polymerisation des zunächst gebildeten Cyanamids gewinnen. Für die Umsetzung wurde die Verwendung eines neutralen wasserfreien Lösungsmittel mit einem Siedepunkt unter 200 °C zwingend vorgeschrieben. Die Polymerisation konnte in Gegenwart oder in Abwesenheit eines Lösungsmittels bei Temperaturen im Bereich von etwa 80 bis 250 °C durchgeführt werden. Wie aus den relevanten Beispielen der US-PS 2,325,586 sowie der US-PS 2,336,605 - Umsetzung von Hexamethylendiamin mit Bromcyan in Ethanol und Polymerisation - hervorgeht, mußte nicht nur ein absolutes organisches Lösungsmittel verwendet werden, sondern die erforderliche Lösungsmittelmenge war auch sehr hoch; die Polymerisation erfolgte durch 25 ständiges Kochen am Rückfluß oder nach Abdestillieren des Lösungsmittels durch 7 ständiges Erhitzen auf 175 °C unter vermindertem Druck. Alle diese Maßnahmen mindern die Wirtschaftlichkeit des Verfahrens.

Aus der US-PS 2,336,605 ist bekannt, die vorgenannten polymeren Guanidinsalze zur Bekämpfung von Bakterien, Pilzen und Insekten einzusetzen. Bei der Untersuchung der mikrobioziden Wirksamkeit von gemäß US-PS 2,336,605 hergestellten Polyhexamethylenguanidinsalzen zeigte sich, daß die Wirksamkeit gegen Staphylococcen nicht befriedigte.

Die Aufgabe der vorliegenden Erfindung bestand somit darin, das gattungsgemäße Verfahren dahingehend zu verbessern, daß sich die Verwendung großer Mengen absoluter Lösungsmittel erübrigt, die Polymerisationszeit verkürzt werden kann und Lösungen polymerer Guanidinsalze mit gegenüber den vorbekannten Lösungen erhöhter Biozidwirksamkeit erhalten werden.

Gefunden wurde nun ein Verfahren zur Herstellung von Lösungen von polymeren Guanidinsalzen mit erhöhter Bakterizidwirksamkeit durch Umsetzung eines Diamins oder Polyamins der allgemeinen Formel (I)

$$H-\underset{\substack{\mid \\ R}}{N}-CH_2-X-CH_2-\underset{\substack{\mid \\ R'}}{N}-H \qquad (I),$$

worin R und R', welche gleich oder verschieden sein können, Wasserstoff oder eine $C_1$ - bis $C_6$-Alkylgruppe bedeuten, und X eine lineare oder verzweigte Alkylengruppe mit 2 bis 16 C-Atomen, welche durch eine oder mehrere isolierte -O- oder -NR" - Gruppen unterbrochen sein kann und worin R" für Wasserstoff oder eine $C_1$ - bis $C_{18}$-Alkylgruppe steht, oder eine 1,2- 1,3- oder 1,4 Cyclohexylen- oder 1,2- oder 1,3-Cyclopentylen mit Chlorcyan im Molverhältnis von im wesentlichen 1 zu 1 in einem organischen Lösungsmittel und anschließend Polymerisation durch Erwärmen des Umsetzungsproduktes in Gegenwart eines organischen Lösungsmittels, das dadurch gekennzeichnet ist, daß man die Polymerisation in einem über 195 °C siedenden, bei 20 °C flüssigen, mehrwertigen Alkohol bei 130 bis 160 °C innerhalb von 15 bis 60 Minuten durchführt.

Weitere Ansprüche richten sich auf bevorzugte Ausführungsformen des Verfahrens sowie auf die nach dem Verfahren erhältlichen Lösungen mit erhöhter Biozidwirksamkeit sowie deren Verwendung zur Herstellung biozider Mittel.

Das erfindungsgemäße Verfahren umfaßt zwei Stufen, nämlich die Umsetzung des Di- oder Polyamins mit Chlorcyan im Molverhältnis von im wesentlichen 1 zu 1 und die Polymerisation des intermediär gebildeten Hydrochlorids des Monocyanamids des Di- oder Polyamins. Überraschenderweise gelingt es, die Biozidwirksamkeit der polymeren Guanidinsalze zu erhöhen, wenn die Polymerisation in Gegenwart eines über 195 °C, vorzugsweise über 200 °C, siedenden mehrwertigen Alkohols, wie beispielsweise Ethylenglykol, Diethylenglykol oder Glycerin durchgeführt wird. Besonders bevorzugt wird Glycerin verwendet. Die in der Polymerisationsstufe zu verwendende Menge mehrwertiger Alkohol, der nicht absolut sein muß, liegt im Bereich von 0,2 bis 5, vorzugsweise 0,5 bis 2, Gew.-teile pro Gew.-teil Di- oder Polyamin.

Beim Aufheizen des Reaktionsproduktes der ersten Stufe des Verfahrens in einem oder einem Gemisch der erfindungsgemäß einzusetzenden mehrwertigen Alkohole, setzt bei 130 bis 140 °C eine deutlich exotherme Reaktion ein. Soweit erforderlich, begrenzt man die Temperatur auf 160 °C. Nach Abklingen der exothermen Reaktion wird die Polymerisation beendet, eine Polymerisationszeit von 15 bis 60 Minuten, vorzugsweise 30 bis 60 Minuten bei einer Temperatur von 130 bis 160 °C, vorzugsweie 130 bis 150 °C ist ausreichend, um Lösungen polymerer Guanidinsalze mit erhöhter Bakterizidwirksamkeit zu erhalten.

Die Umsetzung des Di- oder Polyamins mit Chlorcyan erfolgt in einem organischen Lösungsmittel, wobei sowohl solche gemäß dem vorbekannten Verfahren der US-PS 2,325,568 als auch mehrwertige Alkohole mit einem Siedepunkt über 195 °C, insbesondere 200 °C Verwendung finden können. Es ist nicht nötig, absolute Lösungsmittel einzusetzen. Vorzugsweise wird die Umsetzung bereits in jenem Lösungsmittel durchgeführt, das in der Polymerisationsstufe Verwendung finden soll, Bei Bedarf, etwa um die Viskosität des mehrwertigen Alkohols bzw. Reaktionsgemischs bei niedriger Temperatur zu erniedrigen, kann in der Umsetzungsstufe vorteilhaft zusätzlich ein unter 100 °C siedendes, gegenüber den Reaktanden und dem Reaktionsprodukt unter den Umsetzungsbedingungen - 0 bis 50 °C - inerten und mit dem mehrwertigen Alkohol mischbares organisches Lösungsmittel mitverwendet werden; dieses niedrig siedende Lösungsmittel, vorzugsweise Methanol oder Ethanol, wird vor der Polymerisationsstufe abdestilliert. Die Menge des ggf. mitverwendeten unter 100 °C siedenden Lösungsmittels ist wenig kritsch; üblicherweise wird aber die Menge auf 2 Gew.-teile, vorzugsweise 1 Gew.-teil, pro Gew.-teil Di- oder Polyamin begrenzt.

Die Umsetzung des Di- oder Polyamins mit Chlorcyan erfolgt unter Verwendung von flüssigem oder gasförmigem Chlorcyan bei im allgemeinen 0 bis 50 °C, vorzugsweise 5 bis 20 °C. Ein Molverhältnis von im wesentlichen 1 zu 1 bedeutet, daß ein geringer Überschuß eines Reaktanden möglich ist, sofern sichergestellt wird, daß es bei der Polymerisation zu keiner Vernetzung und damit einem Unlöslichwerden des polymeren Guanidinsalzes kommt.

Gemäß einer bevorzugten Ausführungsform des Verfahrens werden Di- bzw. Triamine der allgemeinen Formel (I) eingesetzt, worin R und R' Wasserstoff bedeuten und X für eine unverzweigte Alkylengruppe mit 4 bis 10 C-Atomen oder eine Diethylenaminogruppe steht. Hexamethylendiamin wird besonders bevorzugt.

Die nach beendeter Polymerisation erhaltenen Lösungen können direkt als biozides Mittel verwendet werden oder zur Herstellung solcher, vorzugsweise in noch warmem Zustand, mit für die Anwendung als Biozid geeigneten Lösungsmitteln wie vorzugsweise Wasser verdünnt und auf die gewünschte Konzentration an Wirkstoff eingestellt werden. Sofern erwünscht, kann das in der erfindungsgemäß hergestellten Lösung enthaltene polymere Guanidinhydrochlorid in an sich bekannter Weise über die Guanidinbase in andere Salze überführt werden.

Die erfindungsgemäß erhaltenen Lösungen weisen eine höhere Biozidwirksamkeit auf als nach dem vorbekannten Verfahren hergestellte Lösungen. Dieser Vorteil zeigt sich insbesondere bei der Wirksamkeit gegenüber Staphylococcen. Zusätzlich zeigen aber die erfindungsgemäßen Lösungen eine bessere, insbesondere raschere Wirkung als Algizid.

Die aus den erfindungsgemäßen Lösungen erhältlichen bioziden Mittel können außer dem polymeren Guanidinsalz und geeigneten Lösungsmitteln auch andere systemverträgliche, biozid wirksame Komponenten und Hilfsstoffe enthalten. Durch die Kombination der erfindungsgemäßen Lösungen mit mehreren strukturähnlichen, z. B. anderen quaternären Ammoniumsalzen oder Guanidiniumalkylbenzolsulfonaten (EP-A 0 338 430), oder strukturverschiedenen Biozidstoffklassen, wie z. B. heterocyclischen Thiocyananten, kann das Wirkungsspektrum erheblich erweitert werden: sofern wasser- und/oder alkoholunlösliche Biozide mitverwendet werden, ist es vorteilhaft, zusätzlich auch Suspensionshilfsmittel einzusetzen.

Die Mittel, welche sich im wesentlichen durch Vereinigen der Komponenten unter Rühren und ggf. Erwärmen herstellen lassen, können in der Wasserbehandlung zur Bekämpfung von Bakterien, Pilzen und Algen, aber auch zur Bekämpfung dieser Keime in technischen Materialien, wie Dispersionen, oder auf Oberflächen eingesetzt werden.

Mittels der Beispiele werden das Verfahren und die biozide Wirksamkeit verdeutlicht.

Beispiel 1

116 g (1,0 Mol) Hexamethylendiamin werden in 100 g Glycerin gelöst und bei 5 bis 10 °C 51 ml (1,0 Mol) Chlorcyan zugetropft. Wegen der hohen Viskosität der Lösung ist eine intensive Durchmischung erforderlich, um die Reaktionswärme abzuführen. Nach beendeter Zugabe wird 30 Minuten bei Raumtemperatur nachgerührt und anschließend auf 140 bis 150 °C hochgeheizt. Bei einer Innentemperatur von etwa 130 °C ist eine deutlich exotherme Reaktion zu beobachten. Man läßt diese abklingen - 40 Minuten bei max. 150 °C - und verdünnt nach Bedarf die viskose Lösung mit Wasser. Die Einhaltung des Temperaturbereiches von 140 bis 150 °C bei der Polymerisation kann im Falle zu starker exothermer Reaktion vorteilhaft durch sog. Siedekühlung vorgenommen werden, indem das siedende Glycerin nach Anlegen eines entsprechenden Vakuums als Kühlmedium dient.

Beispiel 2

Verfahrensweise wie in Beispiel 1, jedoch dient als Lösungsmittel eine Mischung aus 80 g Glycerin und 80 g Ethanol. Nach Zutropfen des Chlorcyans und Nachreaktion wird erwärmt und das Ethanol abdestilliert. Anschließend wird wie in Beispiel 1 bei 140 bis 150 °C polymerisiert. Die Einstellung der Konzentration der Lösung erfolgt durch Zugabe von Wasser.

Beispiel 3

100 g (0,50 Mol) 1,12-Diaminododecan werden in 80 g Glycerin und 100 g Ethanol suspendiert und bei 5 bis 10 °C mit 25,5 ml (0,5 Mol) Chlorcyan versetzt. Man rührt bei 15 °C 30 Minuten nach, destilliert das Ethanol ab und polymerisiert bei 140 °C innerhalb 30 Minuten. Die Lösung kann mit Alkohol verdünnt werden.

Beispiel 4

131,2 g (1,0 Mol) Bis-(3-aminopropyl)-amin werden in 80 ml Diethylenglykol bei 5 bis 10 °C vorgelegt, unter intensivem Rühren 51 ml (1,0 Mol) Chlorcyan zugetropft und 30 Minuten bei 15 bis 20 °C nachgerührt. Man polymerisiert 1 Stunde bei 140 °C und verdünnt mit Wasser.

Beispiel 5

Bestimmung der mikrobioziden Wirkung im Time-Kill-Test

Bei diesem in Anlehnung an die Empfehlungen des American Petroleum Instituts (API, RP 38 2nd ed., Dec. 1965) durchgeführten Test wird eine hoch angereicherte Keimsuspension (Keimzahl $10^6$-$10^8$) mit der gewünschten Menge Biozid versetzt und 24 Stunden bei 25 °C bebrütet. Anschließend wird eine geometrische Verdünnungsreihe bis 6 durchgeführt; je 1 ml wird mit 10 ml Nähragar auf Platten vermischt und 48 Stunden bei 37 °C bebrütet. Aus der Auszahlung der Kolonien wird die Abtötungsrate berechnet.

$$\text{Abtötungsrate in \%} = 100 - \frac{\text{Keimzahl der Probe} \cdot 100}{\text{Keimzahl der Nullprobe}}$$

Die Figur 1/1 zeigt das Verhältnis von Keimzahl zur Abtötungsrate. Hieraus wird deutlich, daß auch eine von 99,9 % auf 100 % gesteigerte Abtötungsrate einer bedeutenden Wirkungsverbesserung entspricht.

Der Tabelle ist die biozide Wirksamkeit (Abtötungsrate (%)) der erfindungsgemäß hergestellten Lösung (Beispiel 1) gegen Bakterien im Vergleich zur bioziden Wirksamkeit der gemäß Beispiel A der US-PS 2,336,605 hergestellten Lösung zu entnehmen. Angegeben ist die Abtötungsrate gegenüber den Testkeimen, wobei die Anwendungskonzentration der Biozide 25 ppm betrug. Die Biozide wurden als 1 gew.-%ige wäßrige, alkoholische Lösung der angereicherten Keimsuspension (40 ml), welche 8,5 g NaCl/l enthielt, zugesetzt.

Tabelle

| Testkeim | Produkt gemäß Beispiel 1 | Produkt gemäß US-PS 2,336,605 |
|---|---|---|
| E. Coli | 100 | 100 |
| Pseudomonas aeruginosa | 100 | 100 |
| Staphylococcus aureus | 99,9 | 93,9 |
| Serratia marcescens | 99,9 | 99,7 |
| Klebsiella pneumoniae | 100 | 100 |

**Patentansprüche**

1. Verfahren zur Herstellung von Lösungen von polymeren Guanidinsalzen mit erhöhter Bakterizidwirksamkeit durch Umsetzung eines Diamins oder Polyamins der allgemeinen Formel (I)

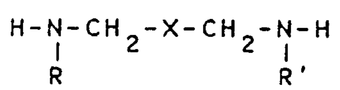

(I),

worin R und R', welche gleich oder verschieden sein können, Wasserstoff oder eine $C_1$- bis $C_6$-Alkylgruppe bedeuten, und X eine lineare oder verzweigte Alkylengruppe mit 2 bis 16 C-Atomen, welche durch eine oder mehrere isolierte -O- oder - NR"- Gruppen unterbrochen sein kann und worin R" für Wasserstoff oder eine $C_1$- bis $C_{18}$-Alkylgruppe steht, oder eine 1,2- 1,3- oder 1,4 Cyclohexylen- oder 1,2- oder 1,3- Cyclopentylen mit Chlorcyan im Molverhältnis von im wesentlichen 1 zu 1 in einem organischen Lösungsmittel und anschließende Polymerisation durch Erwärmen des Umsetzungsproduktes in Gegenwart eines organischen Lösungsmittels,
dadurch gekennzeichnet,
daß man die Polymerisation in einem über 195 °C siedenden, bei 20 °C flüssigen, mehrwertigen Alkohol bei 130 bis 160 °C innerhalb von 15 bis 60 Minuten durch führt.

**2.** Verfahren nach Anspruch 1
dadurch gekennzeichnet,
daß man als mehrwertigen Alkohol Ethylenglykol, Diethylenglykol oder vorzugsweise Glycerin verwendet.

**3.** Verfahren nach Anspruch 1 oder 2,
dadurch gekennzeichnet,
daß man den in der Polymerisationsstufe eingesetzten mehrwertigen Alkohol als alleiniges Lösungsmittel oder im Gemisch mit einem unter 100 °C siedenden organischen Lösungsmittel bereits bei der Umsetzung des Di- oder Polyamins mit Chlorcyan einsetzt, diese Umsetzung bei 0 bis 50 °C durchführt, anschließend das unter 100 °C siedende Lösungsmittel abdestilliert, das Gemisch bis zum Anspringen einer exothermen Reaktion aufheizt und bei 130 bis 150 °C innerhalb von 30 bis 60 Minuten polymerisiert,

**4.** Verfahren nach einem oder mehreren der Ansprüche 1 bis 3,
dadurch gekennzeichnet,
daß man pro Gew.-teil Di- oder Polyamin 0,5 bis 2 Gew.-teile des mehrwertigen Alkohols und, falls zur Viskositätserniedrigung in der Umsetzungsstufe erwünscht, zusätzlich bis zu 2 Gew.-teilen des unter 100 °C siedenden Lösungsmittels, vorzugsweise Methanol oder Ethanol, einsetzt.

**5.** Verfahren nach einem oder mehreren der Ansprüche 1 bis 4,
dadurch gekennzeichnet,
daß man ein Di- oder Triamin der allgemeinen Formel (I) einsetzt, worin R und R' gleich sind und Wasserstoff bedeuten und X für eine unverzweigte Alkylengruppe mit 4 bis 10 C-Atomen oder eine Diethylenaminogruppe steht.

**6.** Verfahren nach Anspruch 5,
dadurch gekennzeichnet,
daß man Hexamethylendiamin verwendet.

**7.** Verwendung der nach den Ansprüchen 1 bis 6 erhältlichen Lösungen polymerer Guanidinsalze zur Herstellung biozider Mittel, insbesondere Bakterizide mit hoher Wirksamkeit gegen Staphylococcen.

**8.** Lösungen polymerer Guanidinsalze mit erhöhter Biozidwirksamkeit, erhalten nach dem Verfahren der Ansprüche 1 bis 6.

**Claims**

**1.** A process for the preparation of solutions of polymeric guanidine salts with increased bactericidal activity by the reaction of a diamine or polyamine of the general formula (I)

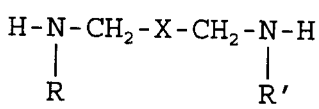

(I),

wherein R and R', which may be the same or different, signify hydrogen or a $C_1$ to $C_6$ alkyl group, and X signifies a linear or branched alkylene group with 2 to 16 C atoms, which may be interrupted by one or more isolated -O- or -NR"- groups and wherein R" denotes hydrogen or a $C_1$ to $C_{18}$ alkyl group, or a 1,2-, 1,3- or 1,4 cyclohexylene or 1,2- or 1,3-cyclopentylene with cyanogen chloride in a molar ratio of essentially 1 to 1 in an organic solvent and subsequent polymerisation by heating the reaction product in the presence of an organic solvent,
characterised in that

the polymerisation is conducted in a polyhydric alcohol which has a boiling point greater than 195°C, and which is liquid at 20°C, within 15 to 60 minutes at 130 to 160°C.

**2.** A process according to claim 1,
characterised in that
ethylene glycol, diethylene glycol or preferably glycerol is used as the polyhydric alcohol.

**3.** A process according to claim 1 or 2,
characterised in that
the polyhydric alcohol used in the polymerisation stage is also used as the sole solvent or mixed with an organic solvent having a boiling point of less than 100°C during the reaction of the diamine or polyamine with cyanogen chloride, this reaction is conducted at 0 to 50°C, the solvent with a boiling point of less than 100°C is then distilled off, the mixture is heated until an exothermic reaction is set up and polymerisation is carried out at 130 to 150°C within 30 to 60 minutes.

**4.** A process according to one or more of claims 1 to 3,
characterised in that
0.5 to 2 parts by weight of the polyhydric alcohol per part by weight diamine or polyamine and, if desired to reduce viscosity in the reaction stage, additionally up to 2 parts by weight of the solvent with a boiling point of less than 100°C, preferably methanol or ethanol, is used.

**5.** A process according to one or more of claims 1 to 4,
characterised in that
a diamine or triamine of the general formula (I) is used, wherein R and R' are the same and signify hydrogen and X denotes an unbranched alkylene group with 4 to 10 C atoms or a diethylene amino group.

**6.** A process according to claim 5,
characterised in that
hexamethylenediamine is used.

**7.** Use of the solutions of polymeric guanidine salts obtainable in accordance with claims 1 to 6 for the preparation of biocidal agents, particularly bactericides with high activity against staphylococci.

**8.** Solutions of polymeric guanidine salts with increased biocidal activity, obtained by the process of claims 1 to 6.

## Revendications

**1.** Procédé de préparation de solutions de sels de guanidine polymères avec une activité biocide accrue par réaction d'une diamine ou polyamine de la formule générale (I) :

$$\begin{array}{ccccccc} H-N & -CH_2-X-CH_2- & N-H \\ | & & | \\ R & & R' \end{array} \qquad (I)$$

dans laquelle R et R', qui peuvent être égaux ou différents, représentent de l'hydrogène ou un groupe alckyle en $C_1$ à $C_6$, et X un groupe alkylène linéaire ou ramifié avec de 2 à 16 atomes de C, qui peut être interrompu par un ou plusieurs groupes isolés -O- ou -NR"- et dans lequel R" représente de l'hydrogène ou un groupe alkyle en $C_1$ à $C_{18}$, ou un 1,2- 1,3- ou 1,4 cyclohexylène ou 1,2- ou 1,3-cyclopentylène, avec du chlorocyanogène dans le rapport molaire essentiellement de 1 à 1 dans un solvant organique et ensuite polymérisation par chauffage du produit de transformation en présence d'un solvant organique, procédé qui est caractérisé en ce qu'on effectue la polymérisation dans un alcool plurivalent bouillant au-dessus de 195°C, liquide à 20°C, entre 130 et 160°C pendant 15 à 60 minutes.

2. Procédé selon la revendication 1, caractérisé en ce qu'on utilise comme alcool plurivalent de l'éthylènglycol, du diéthylènglycol ou de préférence du glycérol.

3. Procédé selon les revendications 1 ou 2, caractérisé en ce qu'on met en oeuvre l'alcool plurivalent utilisé dans l'étape de polymérisation comme solvant unique ou en mélange avec un solvant organique bouillant en dessous de 100°C déjà lors de la réaction de la di- ou polyamine avec du chlorocyanogène, cette réaction est réalisée de 0 à 50°C, ensuite le solvant bouillant en dessous de 100°C est éliminé par distillation, le mélange est chauffé jusqu'au démarrage d'une réaction exothermique et est polymérisé entre 130 et 150°C en 30 à 60 minutes.

4. Procédé selon une ou plusieurs des revendications 1 à 3, caractérisé en ce qu'on met en oeuvre par partie en poids de di- ou polyamine 0,5 à 2 parties en poids de l'alcool plurivalent et, si on le souhaite pour la diminution de viscosité dans l'étape de réaction, en plus jusqu'à 2 parties en poids du solvant bouillant en dessous de 100°C, de préférence du méthanol ou de l'éthanol.

5. Procédé selon une ou plusieurs des revendications 1 à 4, caractérisé en ce qu'on utilise une di- ou triamine de la formule générale (I), pour laquelle R et R' sont égaux et représentent de l'hydrogène et X représente un groupe alkylène non ramifié avec 4 à 10 atomes de C ou un groupe diéthylènamino.

6. Procédé selon la revendication 5, caractérisé en ce qu'on utilise de la héxaméthylèndiamine.

7. Utilisation des solutions de sels de guanidine polymères qu'on obtient selon les revendications 1 à 6 par préparation d'agents biocides, en particulier bactéricides ayant une grande efficacité contre des staphylocoques.

8. Solutions de sels de guanidine polymères ayant une activité biocide élevée, obtenues selon le procédé des revendications 1 à 6.

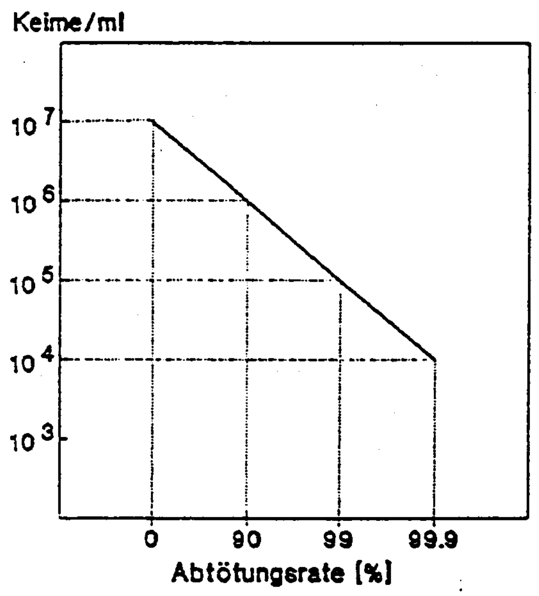
Keime/ml
10 7
10 6
10 5
10 4
10 3
0
90
99
99.9
Abtötungsrate [%]